# EUROPÄISCHE PATENTANMELDUNG

(11) **EP 0 965 313 A1**
(43) Veröffentlichungstag der Anmeldung: **22.12.1999**
(21) Anmeldenummer: 99105893.4
(22) Anmeldetag: 24.03.1999
(51) Int. Cl.: A61F 2/44

(54) **Implantat zur Fusion zweier Wirbel**

(30) Priorität: 17.06.1998 DE 19826619
(71) Anmelder: Ulrich GmbH & Co. KG, 89081 Ulm (DE)
(72) Erfinder: von Strempel, Archibald, 30938 Burgwedel/Thönse (DE)
(74) Vertreter: Hentrich, Swen Dipl.-Phys. Dr.

(57) **Zusammenfassung**

Das Implantat dient zur intersomatischen Fusion zweier benachbarter, posterior zugänglicher Lendenwirbel, wobei es durch zwei Implantatteile (3,4) gebildet ist, bei denen jeweils von einer Basisplatte (5) zwei sich mit gegenseitigem Abstand gegenüberliegende Implantatschenkel (6,7) abstehen, die gemeinsam mit der Basisplatte (5) eine Aufnahme (8) bilden.

## Beschreibung

Die Erfindung betrifft ein Implantat zur intersomatischen Fusion zweier benachbarter Wirbel, insbesondere zweier posterior zugänglicher Lendenwirbel.

Derartige Implantate dienen zur Stabilisierung der Wirbelsäule, nachdem eine als notwendig erachtete Resektion einer Bandscheibe erfolgt ist, die ohne geeignete Maßnahmen zu einem instabilen Segment der Wirbelsäule führt und darüber hinaus infolge des unmittelbaren Kontaktes zweier benachbarter Wirbel Schmerzen verursacht.

Bekannte Implantate stellen sich als weitgehend geschlossene Käfige dar, deren Innenraum nur durch Öffnungen in den Außenwänden zugänglich ist. Durch diese Öffnungen kann der Innenraum mit Knochenspänen oder Knochenzement aufgefüllt werden, um so das Einwachsen des Implantats zu fördern.

Der Erfindung liegt die Aufgabe zugrunde, ein Implantat der eingangs genannten Art so auszubilden, daß das Einwachsen des Implantats durch eine Vergrößerung der Kontaktfläche zwischen Körpergeweben gefördert wird.

Diese Aufgabe wird nach der Erfindung bei einem Implantat der eingangs genannten Art dadurch gelöst, daß es durch ein Implantatteil gebildet ist, bei dem von einer Basisplatte zwei sich mit gegenseitigem Abstand gegenüberliegende Implantatschenkel abstehen, die gemeinsam mit der Basisplatte eine Aufnahme bilden.

Die Erfindung bietet den Vorteil, daß sie ein weitgehend offenes Gerüst zur Verfügung stellt, das die notwendigen statischen Aufgaben übernehmen und die benachbarten Wirbel auf einen vorbestimmten Abstand einstellen kann, wobei das im Querschnitt U-förmige Implantatteil eine große, frei zugängliche Oberfläche bietet, die mit Körpergewebe verwachsen kann. Die Aufnahme hat eine große Öffnungsweite und kann auch Knochenspäne mit größeren Abmessungen aufnehmen. Die große Öffnungsweite fördert auch die Zugänglichkeit der Aufnahme während der Operation. Da ein weitgehend offenes Gerüst vorliegt, ist das Konzept einer biologischen Fusion verwirklicht, bei der überwiegend biologisches, bevorzugt körpereigenes Material am Gerüst gruppiert werden und einwachsen kann. Das Gerüst bietet dem Patienten eine frühe Mobilisierung bei guter Primärstabilität.

Eine besonders bevorzugte Ausführungsform der Erfindung ist dadurch gekennzeichnet, daß zwei der Implantatteile für die gemeinsame Einbringung in den Zwischenwirbelraum vorgesehen sind. Die Verwendung zweier Implantatteile gewährleistet eine gleichmäßige Abstützung der benachbarten Wirbel, auch an weiter entfernt liegenden Stellen der Deckplatten der Wirbel, wobei jedes Implantatteil auf einer Seite des Rückenmarkkanals plaziert ist und nur einen Teil des Zwischenwirbelraumes einnimmt, der so während der Operation zugänglich bleibt und später für die Aufnahme von Knochenspänen zur Verfügung steht.

Wenn die einander abgewandten Implantatschenkel der beiden Implantatteile nach außen zur Senkrechten geneigt von der Basisplatte abstehen, dann ist die Möglichkeit gegeben, daß die Implantatteile sich nicht lediglich auf der Deckplatte der Wirbel abstützen, sondern auch an dem stärker belastbaren Rand der Wirbelkörper, dessen Krümmung die außenliegenden Implantatschenkel infolge ihrer Neigung über eine größere Strecke folgen können.

Zweckmäßigerweise stehen die einander zugewandten Implantatschenkel der beiden Implantatteile rechtwinklig von der Basisplatte ab und verlaufen parallel zueinander. Diese Formgebung erleichtert die Plazierung der Implantatteile im Zwischenwirbelraum beiderseits des Rückenmarkkanals.

Dazu ist im Rahmen der Erfindung weiterhin vorgesehen, daß die beiden Implantatteile mit gegenseitigem Abstand im Zwischenwirbelraum angeordnet sind, auch damit die außenliegenden Implantatschenkel auf dem Rand der Wirbelkörper aufliegen können, ohne daß die Implantatteile ein übermäßig großes Volumen des Zwischenwirbelraumes beanspruchen.

Bei Operationen zur intersomatischen Fusion zweier benachbarter Wirbel hat es sich bewährt, den durch die Implantate eingeschlossenen Raum mit Knochenspänen, Knochenzement, Knochenmehl oder ähnlichem aufzufüllen, um so das Einwachsen des Implantats zu fördern. Im Rahmen der Erfindung ist vorgesehen, daß an den freien Enden der Implantatschenkel Halterungen für eine Verschlußplatte angeordnet sind, wobei die Halterungen durch Stifte gebildet sein können. Hinter diese Stifte kann die Verschlußplatte eingepaßt werden, die die Aufnahme abschließt und dadurch eingebrachtes Material sicher zurückhält.

Besonders bevorzugt ist dabei, wenn die Verschlußplatte durch eine Knochenplatte gebildet ist, die das Einwachsen des Implantats fördert.

Dabei treten dann keine Abstoßungsreaktionen auf, wenn die Knochenplatte aus autologem Knochenmaterial hergestellt ist, wobei der Anteil der Fremdkörper reduziert ist zugunsten der biologischen Fusion.

In den Implantatschenkeln sind Öffnungen ausgebildet, damit der durch die Aufnahme gegebene Innenraum in direktem Kontakt mit dem Außenraum auch im Bereich der Implantatschenkel treten kann, so daß an die Implantatschenkel gelegte Knochenchips durch die Öffnungen hindurch mit den in der Aufnahme eingeführten Knochenspänen oder dergleichen verwachsen können.

Dabei sind die Öffnungen so orientiert, daß die Öffnungen durch zu den benachbarten Wirbeln weisende Zwischenstege getrennt sind, so daß durch die Öffnungen keine Schwächung des Implantats auftritt und die Abstützung der benachbarten Wirbel weiterhin gewährleistet ist.

Um die Implantatteile während der Operation einfach handhaben zu können, ist in der Basisplatte eine Werkzeugaufnahme ausgebildet. Dabei kann die Werkzeugaufnahme als Gewindebohrung ausgestaltet sein, in die ein entsprechendes Werkzeug eingeschraubt wird.

Zur dauerhaften Fixierung der Implantatteile sind an den den Wirbeln zugewandten Seitenrändern der Implantatteile Dorne ausgebildet, die zu einer Verzahnung der Implantatteile mit den Deckplatten der Wirbel führt. Wenn die Dorne zu den Seitenwänden geneigt angeordnet sind, dann tritt zusätzlich noch die Wirkung von Widerhaken hinzu, die eine nachfolgende Lageänderung der Implantatteile verhindert.

Im folgenden soll die Erfindung an einem in der Zeichnung dargestellten Ausführungsbeispiel näher erläutert werden; es zeigen:

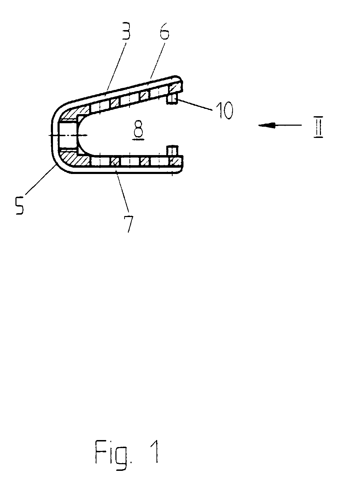

- Fig. 1: einen Querschnitt durch ein Implantatteil,

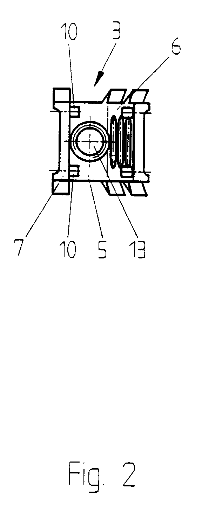

- Fig. 2: die Sicht II aus Fig. 1,

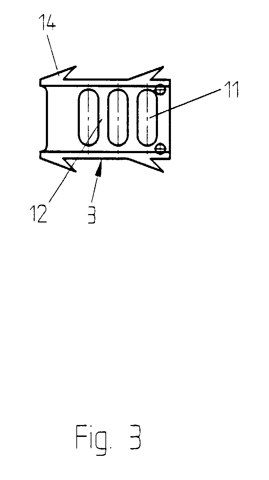

- Fig. 3: eine Seitenansicht des Implantats aus Fig. 2

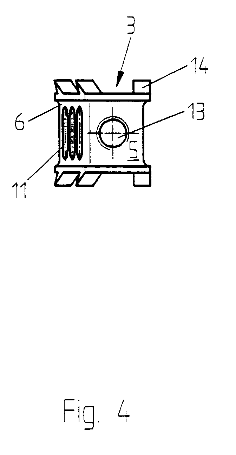

- Fig. 4: eine Rückansicht des Implantats aus Fig. 2,

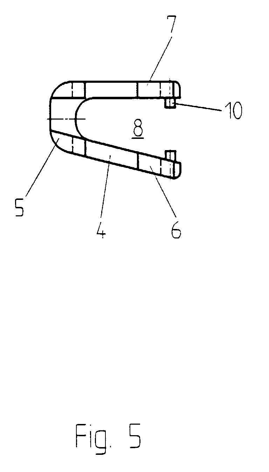

- Fig. 5: eine Draufsicht auf ein zweites Implantatteil,

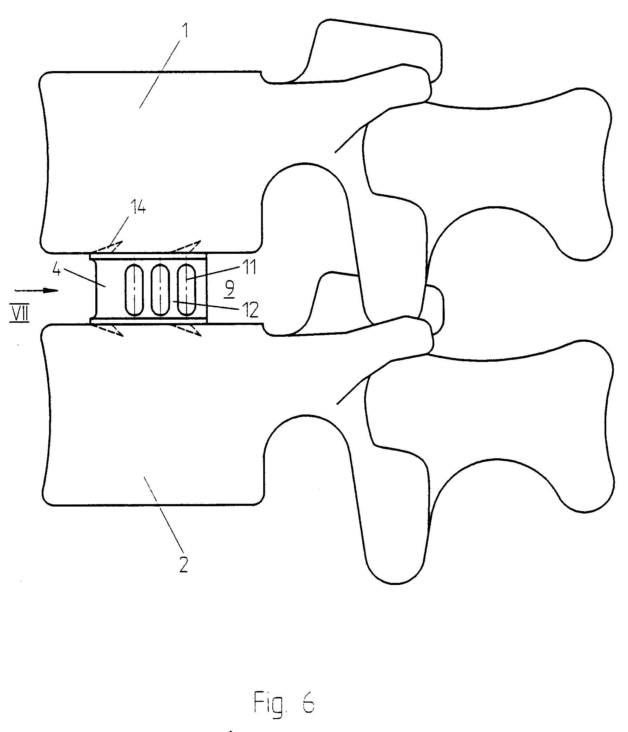

- Fig. 6: eine Seitenansicht eines Zwischenwirbelraum zwischen zwei benachbarten Wirbeln positionierten Implantatteils,

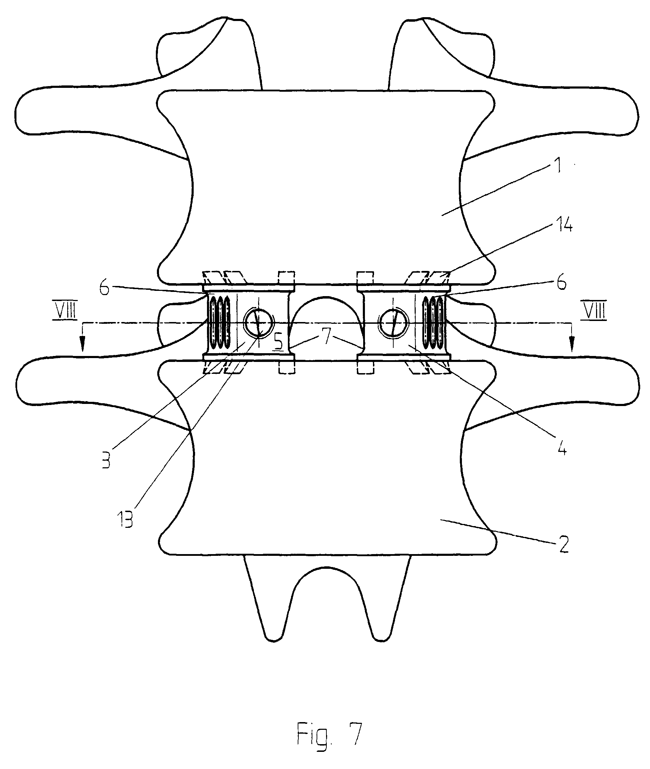

- Fig. 7: die Ansicht VII aus Fig. 6,

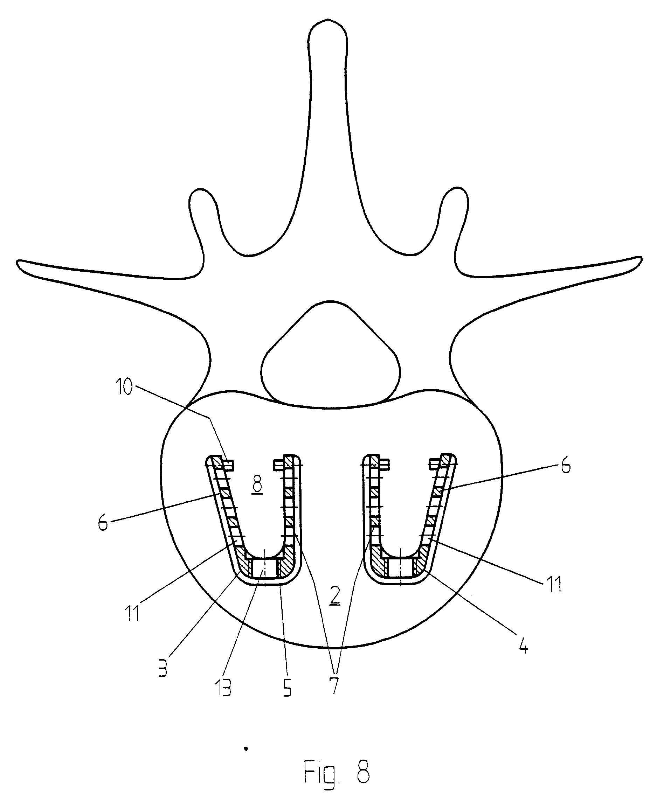

- Fig. 8: der Schnitt VIII-VIII aus Fig. 7.

Das in der Zeichnung dargestellte Implantat dient zur intersomatischen Fusion zweier posterior zugänglicher, benachbarter Lendenwirbel 1,2 und besteht aus zwei paarweise angeordneten Implantatteilen. Jedes Implantatteil 3,4 weist eine Basisplatte 5 auf, von der zwei sich mit gegenseitigem Abstand gegenüberliegende Implantatschenkel 6,7 abstehen, die gemeinsam mit der Basisplatte 5 eine Aufnahme 8 bilden. Dabei sind die einander abgewandten Implantatschenkel 6 des Implantatteilepaars nach außen zur Senkrechten geneigt, während die einander zugewandten Implantatschenkel 7 rechtwinklig von der Basisplatte 5 abstehen und parallel zueinander verlaufen. Bei einer Draufsicht von oben ergibt sich somit eine W-förmige Gestalt der beiden Implantatteile 3,4. Dabei ist jedoch zu beachten, daß die beiden Implantatteile 3,4 im Zwischenwirbelraum 9 mit gegenseitigem Abstand angeordnet sind.

Die offene Aufnahme 8 des Implantats wird durch eine aus autologem Knochenmaterial hergestellte Knochenplatte verschlossen, die durch an den freien Enden der Implantatschenkel 6,7 ausgebildete Halterungen 10 am Implantat fixiert ist. Die Halterungen 10 sind dabei durch Stifte gebildet, hinter die die Knochenplatte gesteckt wird.

In den Implantatschenkeln 6,7 sind Öffnungen 11 ausgebildet, die durch zu den benachbarten Wirbeln 1,2 weisende Zwischenstege 12 getrennt sind. In der Basisplatte 5 ist eine Werkzeugaufnahme 13 vorgesehen, in die während der Operation ein Werkzeug mit korrespondierend geformtem Schaft eingeführt wird. An den den Wirbeln 1,2 zugeordneten Seitenrändern der Implantatteile 3,4 sind Dorne 14 ausgebildet, die nach Art von Widerhaken ausgestaltet sind.

Die Verwendung dieser Implantate ist insbesondere dann vorgesehen, wenn vom Operateur bzw. aus patientenspezifischen Gegebenheiten ein Zugang von posterior erfolgt, bei dem die Implantatteile nach zuvor erfolgter Resektion der Bandscheibe und Distraktion mittels in verschiedenen Größen zur Verfügung stehenden Instrumenten, die alternierend jeweils linksseitig oder rechtsseitig vom Rückenmark eingebracht werden, bis die gewünschte Höhe des intersomatischen Raumes erreicht ist, am Rückenmark vorbei in den Zwischenwirbelraum 9 eingeführt werden, wobei eine Erstlagefixierung durch die Dorne 13 erfolgt. Die Aufnahme 8 wird dann mit Knochenspänen oder ähnlichem Material aufgefüllt und durch die Knochenplatte verschlossen, die aus dem Beckenkamm des Patienten gewonnen werden kann. Um die Implantatteile herum werden Knochenchips plaziert, die durch die in den Implantatschenkeln ausgebildeten Öffnungen 11 mit den Knochenspänen in der Aufnahme verwachsen können, so daß durch das Implantat ein stabiles Gerüst gegeben ist, das infolge des verfolgten Prinzipes, ein möglichst offenes Implantat zur Verfügung zu stellen, ein schnelles Einwachsen des Implantates und damit die Fusion der Wirbel 1,2 ermöglicht. Dabei ist zu beachten, daß die Aufnahme 8 unmittelbar zu den Deckplatten der benachbarten Wirbel 1,2 offen ist und deren volle Grundfläche als Kontaktfläche zur Verfügung steht.

## Patentansprüche

1. Implantat zur intersomatischen Fusion zweier benachbarter Wirbel (1,2), insbesondere zweier posterior zugänglicher Lendenwirbel, dadurch gekennzeichnet, daß es durch ein Implantatteil (3,4) gebildet ist, bei dem von einer Basisplatte (5) zwei sich mit gegenseitigem Abstand gegenüberliegende Implantatschenkel (6,7) abstehen, die gemeinsam mit der Basisplatte (5) eine Aufnahme (8) bilden.

2. Implantat nach Anspruch 1, dadurch gekennzeichnet, daß zwei der Implantatteile (3,4) für die gemeinsame Einbringung in den Zwischenwirbelraum (9) vorgesehen sind.

3. Implantat nach Anspruch 2, dadurch gekennzeichnet, daß die einander abgewandten Implantatschenkel (6) der beiden Implantatteile (3,4) nach außen zur Senkrechten geneigt von der Basisplatte (5) abstehen.

4. Implantat nach Anspruch 2 oder 3, dadurch gekennzeichnet, daß die einander zugewandten Implantatschenkel (7) der beiden Implantatteile (3,4) rechtwinklig von der Basisplatte (5) abstehen und parallel zueinander verlaufen.

5. Implantat nach einem der Ansprüche 2 bis 4, dadurch gekennzeichnet, daß die beiden Implantatteile (3,4) mit gegenseitigem Abstand im Zwischenwirbelraum (9) angeordnet sind.

6. Implantat nach einem der Ansprüche 1 bis 5, dadurch gekennzeichnet, daß an den freien Enden der Implantatschenkel (6,7) Halterungen (10) für eine Verschlußplatte angeordnet sind.

7. Implantat nach Anspruch 6 dadurch gekennzeichnet, daß die Halterungen (10) durch Stifte gebildet sind.

8. Implantat nach Anspruch 6 oder 7, dadurch gekennzeichnet, daß die Verschlußplatte durch eine Knochenplatte gebildet ist.

9. Implantat nach Anspruch 8, dadurch gekennzeichnet, daß die Knochenplatte aus autologem Knochenmaterial hergestellt ist.

10. Implantat nach einem der Ansprüche 1 bis 9, dadurch gekennzeichnet, daß in den Implantatschenkeln (6,7) Öffnungen (11) ausgebildet sind.

11. Implantat nach Anspruch 10, dadurch gekennzeichnet, daß die Öffnungen (11) durch zu den benachbarten Wirbeln (1,2) weisende Zwischenstege (12) getrennt sind.

12. Implantat nach einem der Ansprüche 1 bis 11, dadurch gekennzeichnet, daß in der Basisplatte (5) eine Werkzeugaufnahme (13) ausgebildet ist.

13. Implantat nach einem der Ansprüche 1 bis 12, dadurch gekennzeichnet, daß an den den Wirbeln (1,2) zugewandten Seitenrändern der Implantatteile (3,4) Dorne (14) ausgebildet sind.

14. Implantat nach Anspruch 13, dadurch gekennzeichnet, daß die Dorne (14) zu den Seitenrändern geneigt angeordnet sind.
